# EUROPEAN PATENT APPLICATION

(11) **EP 2 186 512 A1**
(43) Date of publication of application: **19.05.2010**
(21) Application number: 08783597.1
(22) Date of filing: 01.08.2008
(51) Int. Cl.: A61K 31/195, C07C 229/02, C07C 227/16, A61K 9/00, A61K 9/10

(54) **USE OF ALLYLCYSTEINE OR ITS ANALOGS AND PHARMACEUTICAL COMPOSITION THREROF**

(30) Priority: 02.08.2007 CN 200710044525
(71) Applicant: Fudan University, Shanghai 200433 (CN)
(72) Inventor: ZHU, Yizhun, Shanghai 200032 (CN); WANG, Qian, Shanghai 200032 (CN); ZHU, Yichun, Shanghai 200032 (CN); MU, Qing, Shanghai 200032 (CN)
(74) Representative: Crowhurst, Charlotte Waveney
(86) International application number: PCT/CN2008/001408
(87) International publication number: WO 2009/015560

(57) **Abstract**

The invention discloses a use of compound S-allyl cysteine or its analogues in preparation of medicaments for preventing or treating myocardial injury and a preparation method thereof, as well as a pharmaceutical composition comprising the compound.

## Description

### Field of the Invention

The present invention belongs to the field of medical and pharmaceutical technology. The present invention relates to a new medical and pharmaceutical use of S-allyl cysteine compound or its analogues in preparation of medicaments for preventing and/or treating myocardial injury; a use of S-allyl cysteine or its analogues in preparation of medicaments which can improve cell survival rate, produce endogenous H₂S, lower the rate of LDH leakage (LDH release) of myocardial cells injured by hypoxia and hypoglycemia, improve the activity of SOD and/or Catalase and the ability of tissue to inhibit production of hydroxyl free radicals, and inhibit lipid peroxidation and/or myocardial cells apoptosis; a use of S-propargyl cysteine, its pharmaceutically acceptable salts or its solvates in preparation of medicaments which can, by reducing myocardial infarction size, decrease the release of lactate dehydrogenase and creatine kinase, improve the activity of superoxide dismutase, lower the level of malondialdehyde, endogenously produce H₂S and/or inhibit apoptosis; as well as a pharmaceutical composition for preventing and/or treating myocardial injury.

### Background Art

S-allyl cysteine (SAC) is a natural organosulfur compound in garlic, which is the major effective ingredient in aged garlic extract. It has been reported in literature that SAC has antioxidant activity, neuroprotective activity, activity of inhibiting lipid peroxidization induced by excessive oxygen free radicals, and the like.

As reported by literature, there is a certain structure-activity relationship between the allyl structure of SAC and its neuroprotective effect. With regard to the known analogues of SAC, S-ethyl cysteine (SEC), S-propyl cysteine (SPC) and S-allylmercapto cysteine (SAMC) are also natural organosulfur compounds in garlic, and also reported by the literature to have certain antioxidant activities; S-butyl cysteine (SBC) and S-pentyl-cysteine (SPEC) are extended one carbon and two carbons respectively more than the two aforementioned, and is deduced to have similar antioxidant activities; and the compound S-propargyl cysteine is anticipated to have better selectivity to interact with enzyme CSE in that its structure is similar to that of propargylglycine.

However, there has been no report about the effects of SAC on myocardial cells injured by hypoxia and hypoglycemia and on myocardial injury so far.

### Detailed Description of the Invention

To facilitate the understanding of this invention, a plurality of terms are defined below. The terms defined herein have meanings as commonly understood by a person of ordinary skill in the art relevant to the present invention.

Unless otherwise noted, the term "S-allyl cysteine" used herein has the molecular formula and structure as follows:
abbreviation: SAC; molecular formula: C₆H₁₁O₂NS, structural formula:

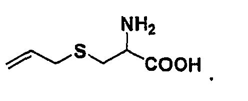

Unless otherwise noted, the term "S-allyl cysteine and its analogues" used herein refers to compounds having structures represented by formula I as follows:

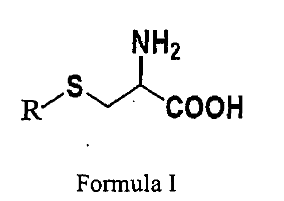

Wherein, R is selected from the group consisting of linear or branched alkyl of C₁-C₅, alkenyl and alkynyl of C₁-C₅, and preferably terminal olefine and terminal alkyne. Because of the central structure of S-allyl cysteine analogues and S-allyl cysteine in common, S-allyl cysteine analogues have similar properties to that of S-allyl cysteine. Wherein, preferred S-allyl cysteine analogues include S-ethyl cysteine (SEC), S-propyl cysteine (SPC), S-allylmercapto cysteine (SAMC), S-butyl cysteine (SBC), S-pentyl -cysteine (SPEC) and S-propargyl cysteine (SPRC), of which the molecular formulae and structures are as follows respectively:
SEC, molecular formula: C₅H₁₁O₂NS, structural formula: 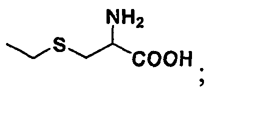
SPC, molecular formula: C₆H₁₃O₂NS, structural formula: 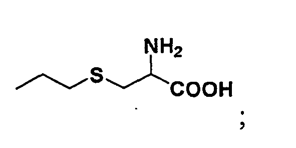
SBC, molecular formula: C₇H₁₅O₂NS, structural formula: 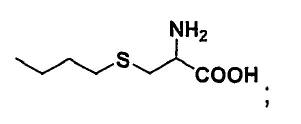
SPEC, molecular formula: C₈H₁₇O₂NS, structural formula: 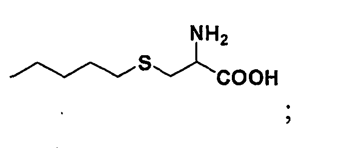
SAMC, molecular formula: C₆H₁₁O₂NS₂, structural formula: 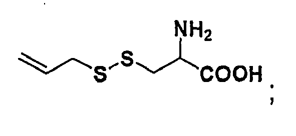
SPRC, molecular formula: C₆H₉O₂NS, structural formula:

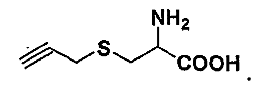

Unless otherwise noted, the term "SOD" used herein refers to superoxide dismutase.

Unless otherwise noted, the term "Catalase" used herein refers to scavenger enzyme.

Unless otherwise noted, the term "LDH" used herein refers to lactate dehydrogenase.

Unless otherwise noted, the term "CK" used herein refers to creatine kinase.

Unless otherwise noted, the term "MDA" used herein refers to lipid peroxide malondialdehyde. Unless otherwise noted, the amount of active ingredient refers to the weight of S-allyl cysteine, or its pharmaceutically acceptable salts or its solvates.

Unless otherwise noted, the term "pharmaceutically acceptable salts" used herein can be understood to be physiologically acceptable salts, especially the salts acceptable to be used as medicaments applied to human and/or mammals.

Unless otherwise noted, the term "pharmaceutically acceptable carrier" used herein refers to those substances publicly known in the art which are used as filling agents or carrier substances in pills, tablets, capsules and the like. These substances are generally recognized by the health-care specialists to be used for this purpose and as non-active ingredients in medicaments. The compilation about pharmaceutically acceptable carriers and excipients can be found in "Handbook of Pharmaceutical excipients" (the second edition edited by A. Wade & P. J. Weller, published by American Pharmaceutical Association ,Washington and The Pharmaceutical Press, London, 1994) and other reference books.

One object of the present invention is to provide a new medical and pharmaceutical use of S-allyl cysteine or its analogues in the preparation of medicaments for preventing and/or treating myocardial injury. Another object of the present invention is to provide a use of S-allyl cysteine or its analogues in preparation of medicaments which can increase cell survival rate, produce endogenous H₂S, reduce LDH leakage of myocardial cells injured by hypoxia and hypoglycemia, improve the activity of SOD and/or Catalase and the ability of tissue to inhibit production of hydroxyl free radicals, inhibit lipid peroxidation and/or inhibit myocardial cells apoptosis. A still another object of the present invention is to provide a use of S-propargyl cysteine, its pharmaceutically acceptable salts or its solvates in the preparation of medicaments which can reduce myocardial infarction size, decrease the release of lactate dehydrogenase and creatine kinase, improve the activity of superoxide dismutase, lower the level of malondialdehyde, endogenously produce H₂S and/or inhibit apoptosis. A yet another object of the present invention is to provide a pharmaceutical composition for preventing and/or treating myocardial injury.

In view of the objects of the invention described above, the technical solution provided in the present invention is as follows:

In one aspect, the present invention provides a use of the compound of the following formula I in the preparation of medicaments for preventing and/or treating myocardial injury: 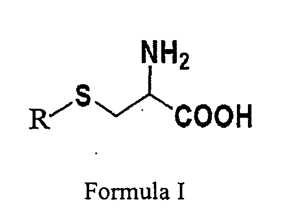 wherein, R is selected from the group consisting of linear or branched alkyl of C₁-C₅, alkenyl and alkynyl of C₁-C₅, and preferably terminal olefine and terminal alkyne; and said compound of formula I is present in the form of the compound itself, its pharmaceutically acceptable salts or its solvates.

Preferably, the heart disease caused by said myocardial injury is one induced by hypoxia and hypoglycemia.

In a further aspect, the present invention provides a use of the compound of formula I as follows in preparation of medicaments which can increase cell survival rate, produce endogenous H₂S, reduce LDH leakage of myocardial cells injured by hypoxia and hypoglycemia, improve the activity of SOD and/or Catalase and the ability of tissue to inhibit production of hydroxyl free radicals, inhibit lipid peroxidation and/or inhibit myocardial cells apoptosis: 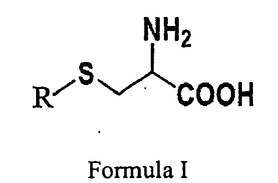 wherein, R is selected from the group consisting of linear or branched alkyl of C₁-C₅, alkenyl and alkynyl of C₁-C₅, and preferably terminal olefine and terminal alkyne; and said compound of formula I is present in the form of the compound itself, its pharmaceutically acceptable salts or its solvates.

In a still further aspect, the present invention provides a use of the compound of formula I as follows, its pharmaceutically acceptable salts or its solvates in preparation of medicaments which can reduce myocardial infarction size, decrease release of lactate dehydrogenase and creatine kinase, improve the activity of superoxide dismutase, lower the level of malondialdehyde, endogenously produce H₂S and/or inhibit apoptosis: 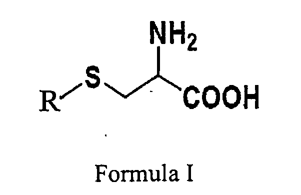 wherein, R is selected from the group consisting of linear or branched alkyl of C₁-C₅, alkenyl and alkynyl of C₁-C₅, and preferably terminal olefine and terminal alkyne.

Preferably, in the use described above of the present invention, said compound of formula I is selected from the group consisting of S-allyl cysteine, S-ethyl cysteine, S-propyl cysteine, S-allylmercapto cysteine, S-butyl cysteine, S-pentyl cysteine and S-propargyl cysteine, preferably S-allyl cysteine and S-propargyl cysteine, and most preferably S-allyl cysteine.

Preferably, in the use described above of the present invention, the dosage form of said medicaments is selected from the group consisting of formulations for oral, parenteral, topical, inhalation and transdermal administration.

In a yet further aspect, the present invention provides a pharmaceutical composition for preventing and/or treating myocardial injury, wherein said pharmaceutical composition comprises:
compound of formula I, its pharmaceutically acceptable salts or its solvates used as active ingredient: 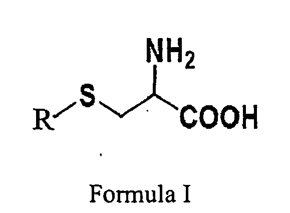
wherein, R is selected from the group consisting of linear or branched alkyl of C₁-C₅, alkenyl and alkynyl of C₁-C₅, and preferably terminal olefine and terminal alkyne;
as well as one or more pharmaceutically acceptable carriers.

Preferably, said solvates of the compound of formula I in the pharmaceutical composition of the present invention are hydrates.

Preferably, in the pharmaceutical composition of the present invention, said compound of formula I is selected from the group consisting of S-allyl cysteine, S-ethyl cysteine, S-propyl cysteine, S-allylmercapto cysteine, S-butyl cysteine, S-pentyl cysteine and S-propargyl cysteine, preferably S-allyl cysteine and S-propargyl cysteine, and most preferably S-allyl cysteine.

Preferably, in the pharmaceutical composition of the present invention, said pharmaceutical composition is selected from the group consisting of oral formulations, formulations for parenteral administration such as injection, and formulations for topical, inhalation and transdermal administration.

Preferably, in the pharmaceutical composition of the present invention, said pharmaceutical composition is selected from the group consisting of oral formulations of tablets, capsules, granules, pills, drops, juice or syrup; preferably, said pharmaceutically acceptable carrier is selected from the group consisting of disintegrating agents, lubricants, binders, filling agents, solvents, flavours, sweetening agents, antioxidants, surfactants, preservatives, correctants and pigments.

In a yet further aspect, the present invention provides a method for preparation of the compound of formula I as follows: 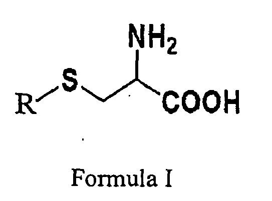 wherein, R is selected from the group consisting of linear or branched alkyl of C₁-C₅, alkenyl and alkynyl of C₁-C₅, and preferably terminal olefine and terminal alkyne; said method comprises steps as follows:
L-cysteine hydrochloride is dissolved in a pre-cooled NH₄OH solution, and Br-R is added to said solution, wherein the substituent R is defined as aforementioned; the mixed solution is stirred at 0-5□ for 1-5 hours and filtered, then the filtrate is distilled in reduced pressure, concentrated and filtered again. The solid separated out is washed repeatedly with ethanol, dried in vacuum and re-crystallized from water/ethanol with a volume ratio from 1: 3 to 3:1.

Now, the technical solution of the present invention will be described in details with reference to a certain number of preferred embodiments related to the invention.

According to one preferred embodiment of the invention, provided is a use of S-allyl cysteine or its analogues in preparation of medicaments for preventing and/or treating myocardial injury, wherein, said S-allyl cysteine or its analogues are present in the form of the compound itself, its pharmaceutically acceptable salts or its solvates.

Preferably, in the use of the present invention, the heart disease caused by myocardial injury is one induced by hypoxia and hypoglycemia.

According to one preferred embodiment of the invention, provided is a use of S-allyl cysteine or its analogues in preparation of medicaments which can increase cell survival rate, produce endogenous H₂S, reduce LDH leakage of myocardial cells injured by hypoxia and hypoglycemia, improve the activity of SOD and/or Catalase and the ability of tissue to inhibit production of hydroxyl free radicals, inhibit lipid peroxidation and/or inhibit myocardial cells apoptosis, wherein, said S-allyl cysteine or its analogues is present in the form of the compound itself, its pharmaceutically acceptable salts or its solvates.

Preferably, in the use of the present invention, said S-allyl cysteine or its analogues are selected from the group consisting of S-ethyl cysteine, S-propyl cysteine, S-allylmercapto cysteine, S-butyl cysteine, S-pentyl cysteine and S-propargyl cysteine.

According to one preferred embodiment of the invention, provided is a use of S-propargylcysteine, its pharmaceutically acceptable salts or its solvates in preparation of medicaments which can reduce myocardial infarction size, decrease the release of lactate dehydrogenase and creatine kinase, increase the activity of superoxide dismutase, lower the level of malondialdehyde, endogenously produce H₂S and/or inhibit apoptosis.

Preferably, in the use of the present invention, the dosage form of said medicaments is selected from the group consisting of formulations for oral, parenteral, topical, inhalation and transdermal administration.

According to one preferred embodiment of the invention, provided is a pharmaceutical composition for preventing and/or treating myocardial injury; said pharmaceutical composition comprises S-allyl cysteine or its analogues as active ingredient and one or more pharmaceutically acceptable carriers, wherein, said S-allyl cysteine or its analogues is present in the form of the compound itself, its pharmaceutically acceptable salts or its solvates.

Preferably, in the pharmaceutical composition of the present invention, said solvates of S-allyl cysteine or its analogues is hydrates.

Preferably, in the pharmaceutical composition of the present invention, said S-allyl cysteine or its analogues is selected from the group consisting of S-ethyl cysteine, S-propyl cysteine, S-allylmercapto cysteine, S-butyl cysteine, S-pentyl cysteine and S-propargyl cysteine.

Further preferably, in the pharmaceutical composition of the present invention, said pharmaceutical composition is selected from the group consisting of oral formulations, formulations for parenteral administration such as injection, and formulations for topical, inhalation or transdermal administration.

Preferably, in the pharmaceutical composition of the present invention, said pharmaceutical composition is selected from the group consisting of oral formulations, such as tablets, capsules, granules, pills, drops, juice or syrup; preferably, said pharmaceutically acceptable carrier is selected from the group consisting of disintegrating agents, lubricants, binders, filling agents, solvents, flavours, sweetening agents, antioxidants, surfactant, preservatives, correctants and pigments.

According to one preferred embodiment of the present invention, the invention provides a method for preparation of S-propargyl cysteine, one of the analogues of S-allyl cysteine, which is **characterized in that** said method comprises steps as follows:
L-cysteine hydrochloride is dissolved in a pre-cooled NH₄OH solution, and 3-propargyl bromide is added to said solution; the mixed solution is stirred at 00 for 2 hours and filtered, then the filtrate is distilled in reduced pressure, concentrated and filtered again. The solid separated out is washed repeatedly with ethanol, dried in vacuum and re-crystallized from water/ethanol with a 2:3 volume ratio to yield white needle crystals.

Said S-propargyl cysteine (SPRC) among analogues of S-allyl cysteine is synthesized through the synthetic route as follows:
L-cysteine hydrochloride is dissolved in a pre-cooled NH₄OH solution (2M, 240ml), and 3-propargyl bromide (14.5g, 0.124 mol) is added to said solution; the mixed solution is stirred at 0□ for 2 hours and filtered, then the filtrate is concentrated in reduced pressure ( <400 ) to a smaller volume, and filtered again. The solid separated out is washed repeatedly with ethanol, dried in vacuum and re-crystallized from water/ethanol with a 2:3 volume ratio to yield white needle crystals. It is determined by detecting NMR structure with Proton Nuclear Magnetic Resonance Spectroscopy.

The present inventors have conducted experiments in vivo and in vitro as followes on the S-allyl cysteine compounds described above, and thereby sufficiently validated the exploitativeness of the medical and pharmaceutical use of the present invention.

By the experiment on hypoxia and hypoglycemia myocardial cells, the present invention has detected the supernatant of hypoxia and hypoglycemia myocardial cells treated by SAC and its analogues, of which the results showed that there was a significant increase in the concentration of H₂S, demonstrating that SAC and its analogues can produce endogenous H₂S in vivo.

By the culturing experiments on myocardial cells in vitro, the present invention has proved that SAC and its analogues can increase cell survival rate, produce endogenous H₂S; reduce the LDH leakage of myocardial cells injured by hypoxia and hypoglycemia; improve the activity of SOD and Catalase, and the ability of tissue to inhibit production of hydroxyl free radicals; inhibit lipid peroxidation and inhibit myocardial cells apoptosis, demonstrating that said S-allyl cysteine and its analogues can be used as treatment medicaments for treating myocardial injury, especially for treating heart diseases caused by hypoxia and hypoglycemia-induced myocardial injury.

Methods known in the art may be used to prepare the composition of the present invention, preferably in the form of formulations. Such methods comprise a step of combining the active ingredients with carriers which constitute one or more adjuvant ingredients. Such adjuvant ingredients include those ingredients generally used in the art, such as filling agents, binders, diluent agents, disintegrating agents, lubricants, coloring agents, flavoring agents and wetting agents.

Formulations suitable for oral administration may be prepared into dispersible units such as pills, tablets or capsules that contain predetermined amounts of active ingredients, and prepared into powder or granules, and solution or suspension. The active ingredients may be also prepared into boluses or paste or contained in liposomes.

Formulations for rectal administration may be prepared into suppositories or enemas.

With regard to parenteral administration, suitable formulations include aqueous and non-aqueous sterile injections (the purity of active compounds ≥90%). Such formulations may be prepared into the form of unit dosage or multi-dosage containers such as sealed vials and ampoules, and such formulations may be stored under the condition of freeze-drying (lyophilization) where only sterile solution, such as water, is required to be added before use.

Formulations suitable for administration through nasal inhalation include fine power or spray which may be produced by compressing aerosols, sprays and inhalers with batching dosages.

In order to formulate dosage units such as tablets, what should be concerned is the application of generally used additives, such as filling agents, coloring agents and polymerisable binders and so on. Generally speaking, any pharmaceutically acceptable additives which will not interfere with the function of active compounds may be used.

Suitable additives and/or carriers in the disclosure of the present invention are all the materials used to obtain herbal formulations known by the skilled person from the prior art. The selection and the amounts of these carriers depend on whether the drug is administered orally, intravenously, intraperitoneally, intradermal, intramuscularly, nasally, buccally or topically. Formulations in the forms of tablets, chewable tablets, coating tablets, capsules, granules, drops, juice or syrup are suitable for oral administration, whereas solutions, suspensions, easily reconstituted dried formulations and spraying formulations are suitable to be used in parenteral, topical and inhalation administration. Suppository for rectal administration may also be used. Carrier film or patch may be stored and administrated in the form of dissolution optionally in conjunction with other agents that facilitate percutaneous absorption, which are exemplary examples of suitable transdermal formulations.

Examples of carriers and additives in the formulations for oral administration include disintegrating agents, lubricants, binders, filling agents, and optionally include solvents, flavours, flavoring agents, and especially carrier substances, diluent agents, dyes and antioxidants etc. Wherein, with regard to suppository, wax and fatty acid wax may be used, and as for ingredients for parenteral administrations, carrier substances, preservatives, suspending agents and the like may be used. The amounts of active substances administered to patients vary depending on the functional relationship constituted by the body weight of the patient, forms of administration and extent of the disease. The compound according to the present invention can be released in a sustained release form from the formulation for oral, rectal or transdermal administration. Corresponding sustained release formulation, especially the formulations of "once per day" which only need taking one time per day are particularly suitable for indications according to the present invention.

The carriers of formulations for oral administration of the present invention may be, for example, water, ethanol, isopropanol, propanetriol, glycol, propanediol, polyethylene glycol, polypropylene glycol, glucose, fructose, lactose, saccharose, syrup, starch, modified starch, glutin, sorbitol, inositol, mannitol, avicel, methylcellulose, carboxymethyl cellulose, cellulose acetate, shellac, cetol, polyvinylpyrrolidone, parrafin, wax, natural and synthesized rubber, arabic gum, alginate, dextran, saturated fatty acid and unsaturated fatty acid, stearic acid, magnesium stearate, zinc stearate, glycerin stearate, sodium lauryl sulfate, edible oil, sesame oil, cocoanut oil, peanut oil, soybean oil, lecithin, sodium lactate, polyoxyethylene fatty acid ester and polyoxypropylene fatty acid ester, sorbitan fatty acid ester, sorbic acid, benzoic acid, citric acid, ascorbic acid, tannic acid, sodium chloride, potassium chloride, magnesium chloride, calcium chloride, magnesium oxide, zinc oxide, silica, titanium oxide, titanium dioxide, magnesium sulfate, zinc sulfate, calcium sulfate, potassium carbonate, calcium phosphate, dicalcium phosphate, potassium bromide, potassium iodide, talc, white bole, colloid, Crospovidone, agar and bentonite.

Medicaments and ingredients of medicaments according to the present invention may be prepared with the aid of the technologies described for medicament formulation, such as the known advanced means, facilities, methods and procedures. For example, for solid formulations, such as tablets, the active ingredients in the medicaments, i.e. S-allyl cysteine or its pharmaceutically acceptable salts or its solvates, may be granulated with medicament carriers together, for example, the conventional ingredients in tablets, such as corn flour, lactose, saccharose, sorbitol, talc, magnesium stearate, dicalcium phosphate or colloid pharmaceutically acceptable, to yield one type of solid ingredient containing uniformly distributed S-allyl cysteine or its pharmaceutically acceptable salts or its solvates.

"Uniformly distributed" used herein may be understood as that S-allyl cysteine or its pharmaceutically acceptable salts or its solvates, which are the active ingredient, are uniformly distributed in the whole ingredient, so as to be easily divided into unit dosage having the same activity, such as tablets, pills or capsules. Then the solid ingredient is divided into unit dosage. The tablets or pills of the medicaments or the ingredients of medicaments according to the present invention may also be coated in or mixed with another ingredient to provide a sustained release dosage. Wherein, the suitable coating ingredients are acidized polymers and mixtures of acidized polymers with materials such as shellac, cetol and/or cellulose acetate.

The pharmaceutical compositions of the present invention may be prepared into clinically acceptable formulations, such as tablets, capsules, granules, oral liquid formulations, subcutaneous administration formulations and suppositories and so on. Suitable carriers may be added so as to prepare formulations for oral administration, such as tablets, granules or capsules. These dosage forms are prepared according to the methods well known to those skilled in the art. The carriers used in moulding processes for formulationg tablets, granules or capsules and etc. are auxiliary agents in common use, such as starch, glutin, arabic gum, polyethyleneglycol and the like, and in addition surfactants, lubricants, disintegrating agents, preservatives, correctants, pigments and the like.

### Brief Description of the Drawings

The examples of the present invention will be set forth in details below by making reference to the figures, wherein:
Figure 1 is the column diagram showing effects of SAC and its analogues on survival rate of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05.
Figure 2 is the column diagram showing effects of SAC and its analogues on H₂S release of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05.
Figure 3 is the column diagram showing effects of SAC and its analogues on LDH leakage of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,p < 0.05.

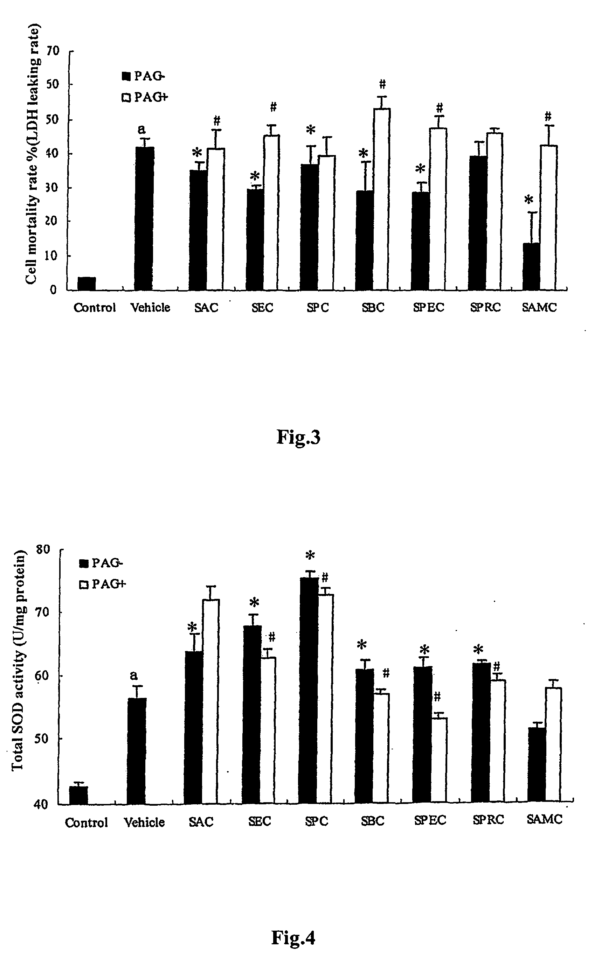

Figure 4 is the column diagram showing effects of SAC and its analogues on total SOD activity of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,*p* < 0.05.

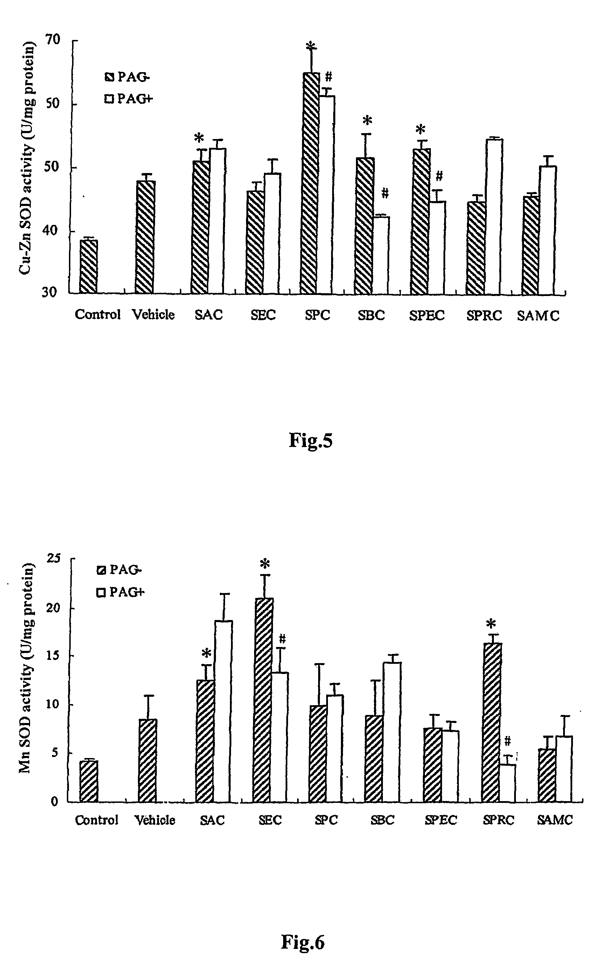

Figure 5 is the column diagram showing effects of SAC and its analogues on Cu-Zn SOD activity of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,p < 0.05.
Figure 6 is the column diagram showing effects of SAC and its analogues on Mn SOD activity of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,p < 0.05.

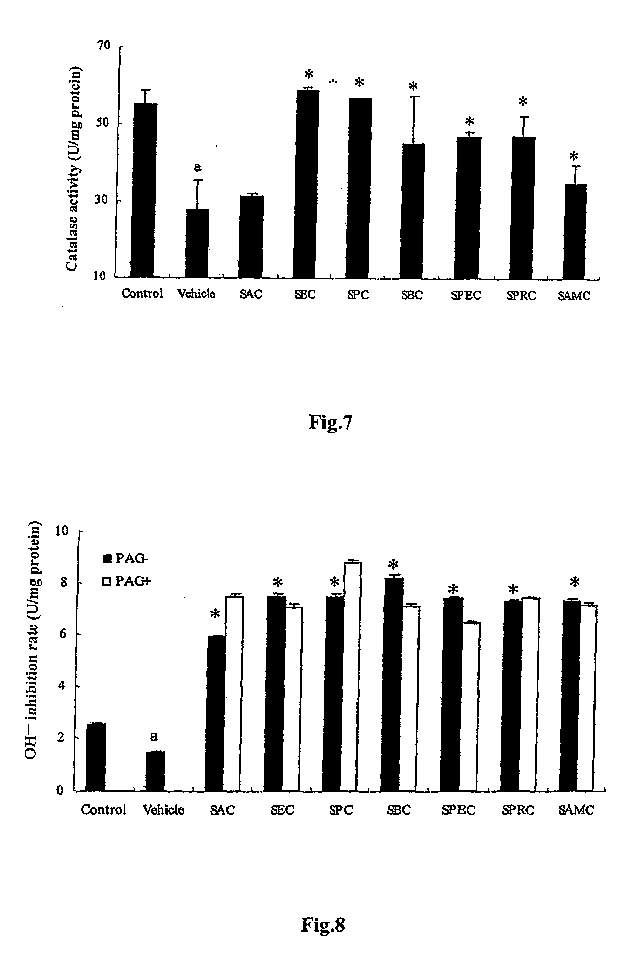

Figure 7 is the column diagram showing effects of SAC and its analogues on catalase activity of myocardial cells injured by 6-hour of glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05.
Figure 8 is the column diagram showing effects of SAC and its analogues on hydroxyl free radical inhibition rate of myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,p <0.05.

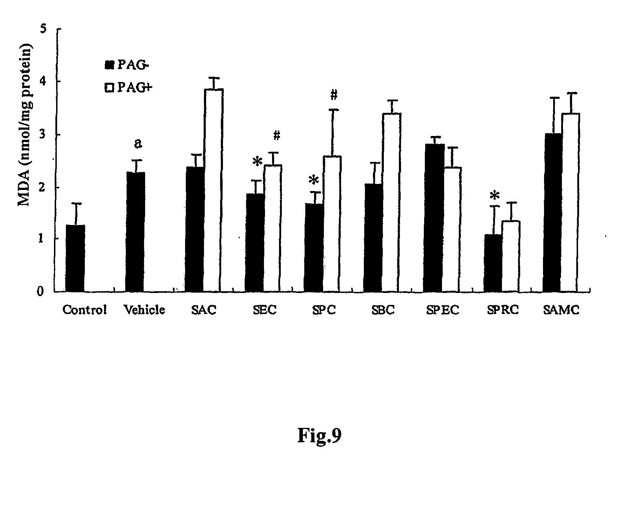

Figure 9 shows effects of SAC and its analogues on MDA in myocardial cells injured by 6-hour glucose and oxygen deprivation / 3-hour glucose and oxygen reintroduction;
   wherein, Control is normal control group; Vehicle is model group; a: comparison of model group to normal control group, *p* <0.05; *: comparison of groups subjected to SAC and its analogues administration to model group, *p* <0.05; #: comparison of groups subjected to SAC and its analogues administration to PAG (propargylglycine, available from Shanghai Intechem Technology Co., Ltd.) intervention group,p < 0.05.

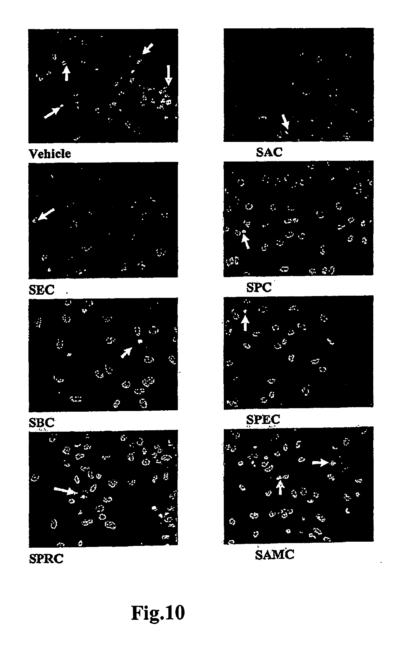

Figure 10 is the electron microscope staining image showing effects of SAC and its analogues on apoptosis of hypoxia and hypoglycemia injured myocardial cells, wherein, what the arrows point to is apoptotic cell nuclei which is pyknotic and stained dark.

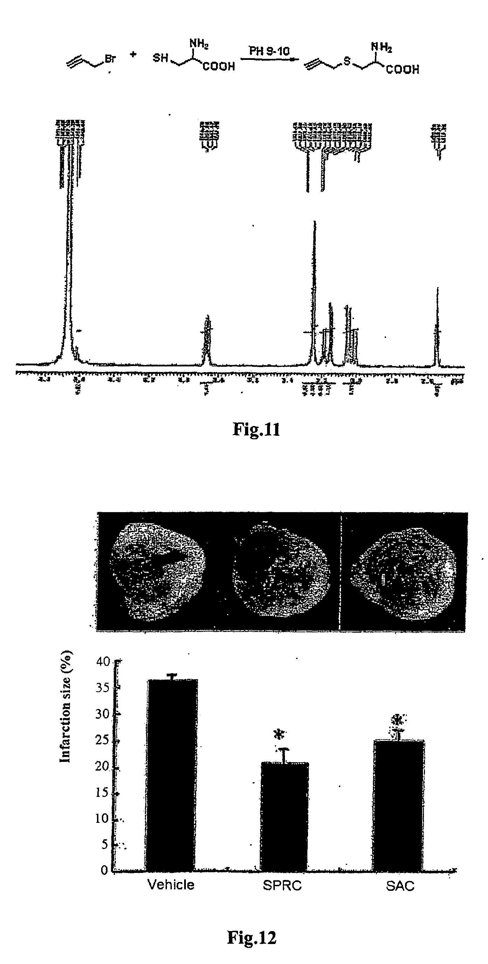

Figure 11 shows synthetic route of SPRC and its structure determined by Proton Nuclear Magnetic Resonance Spectroscopy.
Figure 12 shows effects of SPRC and SAC on infarction size in rats with myocardial infarction, wherein, Vehicle is MI model group; *: comparison to MI model group,p < 0.01;

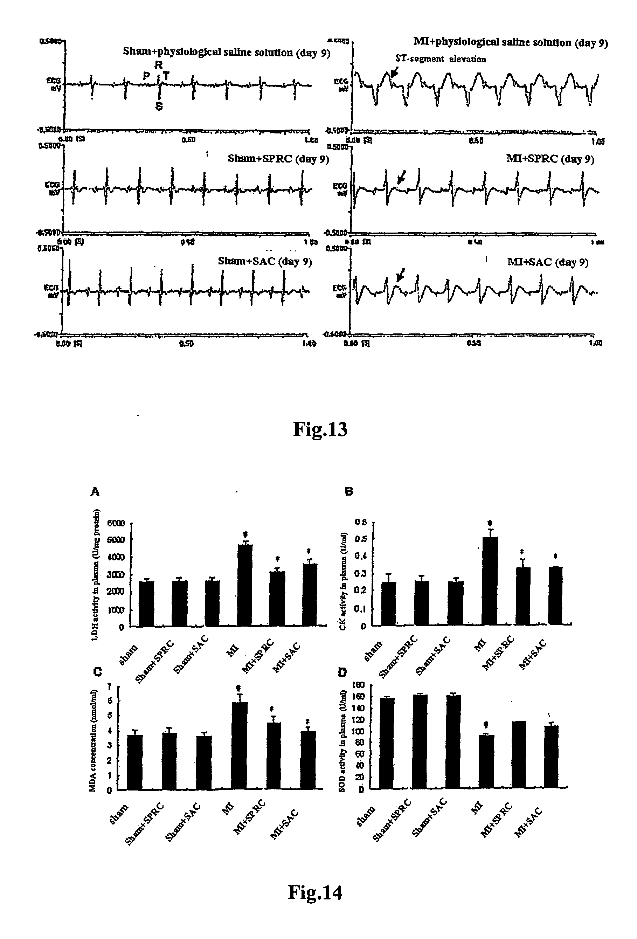

Figure 13 shows effects of SPRC and SAC on cardiogram of rats with myocardial infarction;
   wherein, Sham is sham operated group; Sham +SPRC is sham operated + SPRC (50mg/kg) group; sham + SAC is sham operated + SAC (50mg/kg) group; MI is myocardial infarction model group; MI + SPRC is model + SPRC (50mg/kg) group; MI + SAC is model + SAC (50mg/kg) group.
Figure 14 shows effects of SPRC and SAC on serum LDH, CK, MDA and SOD in rats with myocardial infarction;
   wherein, Sham is sham operated group; Sham +SPRC is sham operated + SPRC (50mg/kg) group; Sham + SAC is sham operated + SAC (50mg/kg) group; MI is myocardial infarction model group, MI + SPRC is model + SPRC (50mg/kg) group; MI + SAC is model + SAC (50mg/kg) group; *: comparison to MI model group, *p* <0.01; #: comparison of MI group to Sham group, p<0.01.

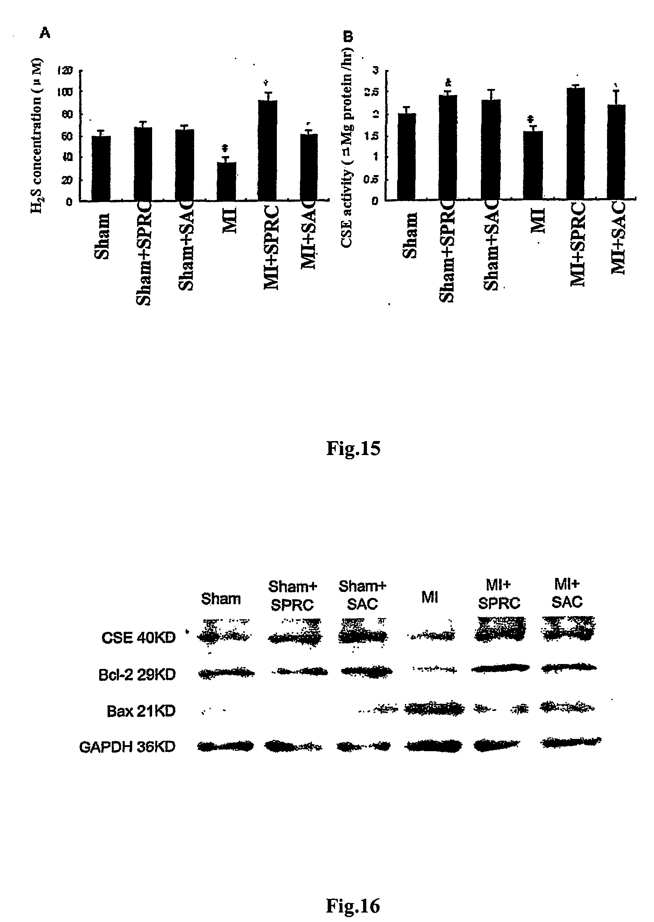

Figure 15 is a column diagram showing effects of SPRC and SAC on H₂S level of serum and CSE activity of cardic tissue in rats with myocardial infarction;
   wherein, Sham is sham operated group; Sham +SPRC is sham operated + SPRC (50mg/kg) group; Sham + SAC is sham operated + SAC (50mg/kg) group; MI is myocardial infarction model group; MI + SPRC is model + SPRC (50mg/kg) group; MI + SAC is model + SAC (50mg/kg) group; *: comparision of groups subjected to SPRC and SAC administration to model group, *p* < 0.01; #: comparison of MI group to Sham group, *p*<0.01.
Figure 16 is the protein electrophoretogram showing effects of SPRC and SAC on protein expressions of Bcl-2, Bax and CSE in left ventricle of rats with myocardial infarction;
   wherein, Sham is sham operated group; Sham +SPRC is sham operated + SPRC (50mg/kg) group; Sham + SAC is sham operated + SAC (50mg/kg) group; MI is myocardial infarction model group; MI + SPRC is model + SPRC (50mg/kg) group; MI + SAC is model + SAC (50mg/kg) group.

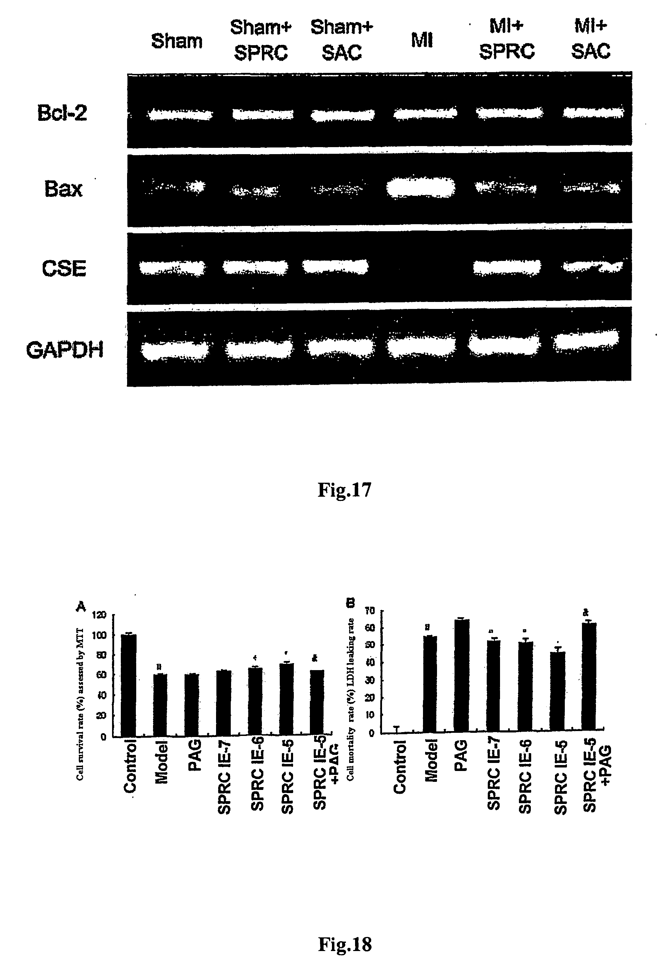

Figure 17 is the protein electrophoretogram showing effects of SPRC and SAC on gene expression of Bcl-2, Bax and CSE in left ventricle of rats with myocardial infarction;
   wherein, Sham is sham operated group; Sham +SPRC is sham operated + SPRC (50mg/kg) group; sham + SAC is sham operated + SAC (50mg/kg) group; MI is myocardial infarction model group; MI + SPRC is model + SPRC (50mg/kg) group; MI + SAC is model + SAC (50mg/kg) group.
Figure 18 is the column diagram showing effects of different dosages of SPRC on cell survival rate and injury extent of H9c2 cells injured by H₂O₂;
   wherein, Control is normal control group; Model is model group; PAG ( propargylglycine, available from Shanghai Intechem Technology Co., Ltd.): PAG 10⁻⁴mol/L; SPRC 1E-7: SPRC 10⁻⁷mol/L; SPRC 1E-6: SPRC 10⁻⁶mol/L; SPRC 1E-5: SPRC 10⁻⁵mol/L; SPRC+PAG: SPRC 10⁻⁵mol/L+PAG10⁻⁴mol/L; #: comparison of model group to normal control group, *p*<0.01; *: comparison of SPRC different dosages groups to model group, *p*<0.05; &: comparison of SPRC + PAG group to SPRC high dosage group.

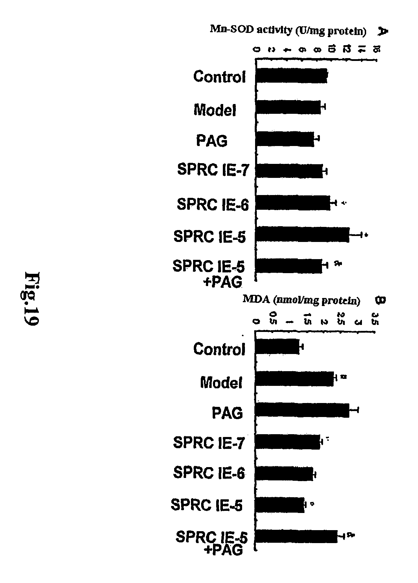

Figure19 is a column diagram showing effects of different dosages of SPRC on Mn-SOD activity and MDA content of H9c2 myocardial cells injured by H₂O₂;
   wherein, Control is normal control group; Model is model group; PAG ( propargylglycine, available from Shanghai Intechem Technology Co., Ltd.): PAG 10⁻⁴mol/L; SPRC 1E-7: SPRC 10⁻⁷mol/L; SPRC 1E-6: SPRC 10⁻⁶mol/L; SPRC 1E-5: SPRC 10⁻⁵mol/L; SPRC+PAG: SPRC 10⁻⁵mol/L+PAG10⁻⁴mol/L; #: comparison of model group to normal control group, *p* < 0.01; *: comparison of SPRC different dosages groups to model group,*p*<0.05; &: comparison of SPRC + PAG group to SPRC high dosage group, p<0.05.

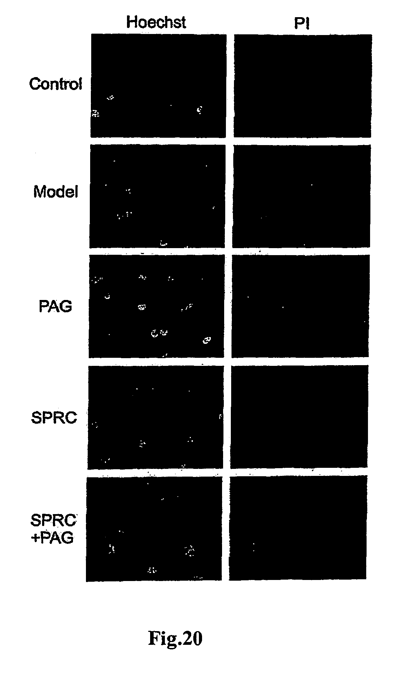

Figure 20 is the electron microscope image showing effects of SPRC on apoptosis of myocardial cells Hgc2 injured by H₂O₂;
   wherein, Control is normal control group; Model is model group; PAG: PAG 10⁻⁴mol/L; SPRC: SPRC 10⁻⁵mol/L; SPRC+PAG: SPRC 10⁻⁵mol/L+PAG10⁻⁴mol/L.

### Best Modes for Carrying Out the Invention

Herein the present invention will be further illustrated by making reference to particular examples. However, these examples are only limited to describing the present invention, and are not used to limit the scope of the present invention. Experimental methods not given specific experimental conditions are generally in conformity with conventional conditions or conditions suggested by manufacturers.

### Example 1: experiments to validate effects of SAC and its analogues on increasing survival rate of cells and producing endogenous H₂S.

A heart sample of 3-day-old SD suckling rat of primary culture was taken through conventional methods under sterile conditions. The heart sample was washed in PBS, treated, put into a flask containing 0.08% trysin solution and digested under the temperature of 37□ for 10 minutes. The digesting process was repeated 8 times wherein the solution was magnetically stirred constantly. The supernatant of each digestion was collected, and then serum was added to stop digestion. The supernatant was centrifuged at 2000 rpm for 5 minutes and collected the cell sediments. The cell density was adjusted to 10⁻⁶, cultured in DMEM containing 10% fetal bovine serum and was used in the experiment at day 3.

The cultured cells described above were divided randomly into following groups:
normal control group: no medicaments intervention, no glucose and oxygen deprivation;
model group: no medicaments intervention, 6-hour glucose and oxygen deprivation / glucose and oxygen reintroduction;
groups subjected to SAC and its analogues: subjected to 10⁻⁵mol/L medicaments administration respectively, glucose and oxygen deprivation / glucose and oxygen reintroduction.

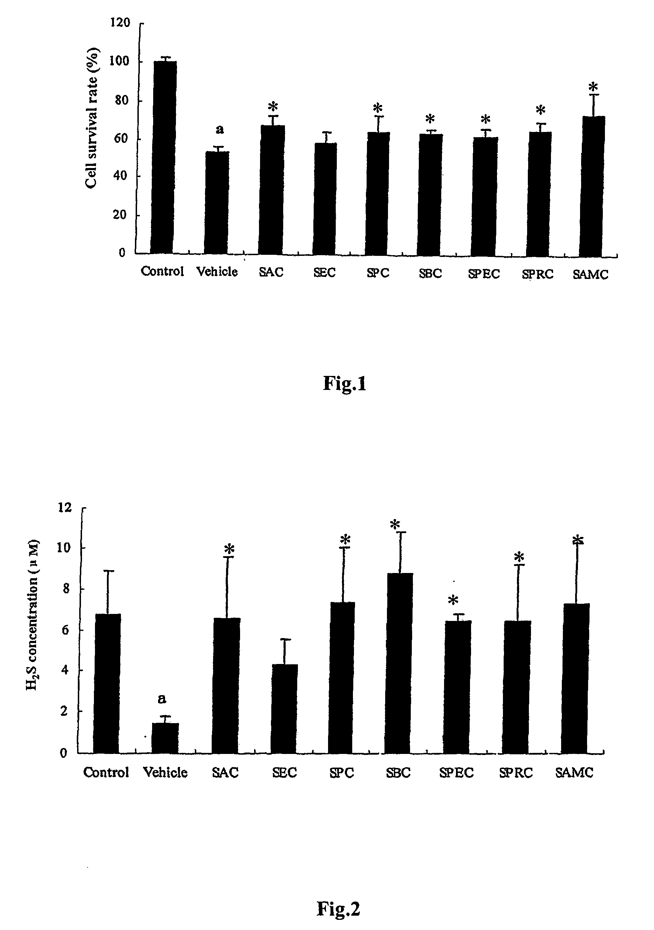

The cell survival rate was assessed by MTT method. As shown in Figure 1, the results illustrate that the survival rate in model group was 54.35%, obviously lower than that in normal control group, and the SAC and its analogues can obviously increase cell survival rate with one way analysis of variance *p*<0.05.

500µl of cell supernatant from each group was taken and added 250µl inc acetate, 133µl of 20mM N, N-dimethyl-phenylenediamine hydrochloride and 133µl of 30mM FeCl₃. The system was shaken thoroughly and then was made to react under room temperature for 10 minutes. After reaction, 10 % trichloroacetic acid was added to precipitate protein. The system was centrifuged 10,000g for 10 minutes , and the absorbance value of each system was measured at 670 nm . As shown in Figure 2, the results illustrate that the H₂S concentration in model group was obviously decreased compared with that in normal control group, with one way analysis of variance *p*<0.05, and SAC and its analogues both can obviously increase the H₂S concentration in the supernatant (*p*<0.05) .

### Example 2: experiments to validate effects of SAC and its analogues on reducing LDH leakage from myocardial cells injured by hypoxia and hypoglycemia

This example is shown in Figure 3.

The myocardial cells of suckling rat were primarily cultured. The model was established by 6-hour glucose and oxygen deprivation / glucose and oxygen reintroduction. The cells were divided into normal control group, model group and groups subjected to SAC and its analogues, which is the same as that in Example 1.

10⁶ cells were obtained from each sample of each group (n=4). Then the cells were lysed thoroghly and their LDH contents were tested by pyruvic acid method. The LDH content in normal control group was 100%. The difference between the LDH in cells of normal control group and of each group subjected to medicament administration and model group was the LDH leaking rate, which was calculated per unit/mg proteins with 1µmol pyruvic acid produced in the reaction system as 1 unit. H₂S production blocking agent PAG was applied to each group subjected to medicament administration parallely to block H₂S production. As shown in Figure 3, the results illustrate that LDH leaking rates in each group subjected to medicament administration were significantly lowered compared with that in model group, and PAG can significantly block the production of SAC and its analogues, indicating that the protective effect of SAC and its analogues on heart and blood vessels was correlated with the endogenous production of H₂S in part.

### Example 3: experiments to validate effects of SAC and its analogues on improving the activity of SOD and Catalase and improving the ability of tissue to inhibit the production of hydroxide free radicals

This example is shown in Figures 4-8.

The myocardial cells of suckling rat were primarily cultured. The model was established by 6-hour glucose and oxygen deprivation / glucose and oxygen reintroduction. The cells were divided into normal control group, model group and groups subjected to SAC and its analogues, just as in Example 1.

SOD was determined by Hydroxylamine method, where 1 unit of SOD activity (U) is, the amount of SOD used in the reaction when the SOD activity is inhibited to its 50 % of original activity by each microgram of tissue protein in 1 milliliter reaction solution. The results (Figure 4) show that total SOD activity can be obviously improved by all SAC and its analogues, and significantly increased compared with that in model group (*p*<0.05). SOD subtype tests reveal that SPC, SBC and SPEC which are of saturated carbon chains mainly elevate the activities of Cu-Zn SOD in cytoplasm (Figure 5) and can be blocked by PAG; whereas new compounds SPRC and SEC mainly elevate the Mn SOD activity in mitochondria (Figure 6 )and can be also blocked by PAG, indicating that SAC and its analogues can obviously improve SOD activity, of which the effects were correlated with H₂S production in part.

The excessive hydrogen peroxide remained from Catalase catalyzation can oxidize chromogenic substrate with the catalyzation of Catalase, to produce red colored product (N-(4-antipyryl)-3-chloro-5-sulfonate-p-benzpquinonemonoimine) with a maximum absorption wavelength at 520nm. One enzyme activity unit (U) can catalyze the decomposition of 1µmol hydrogen peroxide at 25□, pH 7.0 in 1 minute. The results (Figure 7) show that the Catalase activity in model group was significantly lowered compared with that in normal control group (*p*<0.05), indicating that all SAC and its analogues can elevate Catalase activity than model group (*p*<0.05).

The amount of OH⁻ was detected in Fenton reaction. The amount of H₂O₂ was proportional to the amount of OH⁻ produced by Fenton reaction. The H₂O₂ was given electron receptor, and then reacted with gress agent to produce red colored substance, which was detected at a wavelength of 550nm. The results (Figure. 8) show that the hydroxide free radical inhibition rates in groups subjected to SAC and its analogues administration were significantly elevated (*p*<0.05), validating that SAC and its analogues can significantly inhibit the production of OH⁻. The inhibition effects of SAC and its analogues on hydroxide free radicals can not be blocked by PAG.

### Example 4: experiments to validate effects of SAC and its analogues on inhibiting lipid peroxization

This example is shown in Figure 9.

The myocardial cells of suckling rat were primarily cultured. The model was established by 6-hour glucose and oxygen deprivation / glucose and oxygen reintroduction. The cells were divided into normal control group, model group and groups subjected to SAC and its analogues, just as in Example 1.

Oxygen free radical attacks poly unsaturated fatty acid in biomembrane, inducing lipid peroxidation and therby producing lipid peroxide malondialdehyde (MDA). MDA can condensate with thiobarbituric acid (TBA) and form a red product, which has a maximum absorption peak at 532 nm. The results (Figure 9) show that the MDA content in model group was significantly increased compared with that in normal control group (*p*<0.05). The MDA contents in groups subjected to SEC, SPC and SPRC administration were all significantly lowered compared with that in model group, validating that SEC, SPC and SPRC can inhibit lipid peroxidation induced by active oxygen, and these effects can be blocked by PAG, wherein the effect of compound SPRC on inhibiting lipid peroxidation was more significant.

### Example 5: experiments to validate effects of SAC and its analogues on inhibiting myocardial cells apoptosis

This example is shown in Figure 10.

The myocardial cells of suckling rat were primarily cultured. The model was established by 6-hour glucose and oxygen deprivation / glucose and oxygen reintroduction. The cells were divided into normal control group, model group and groups subjected to SAC and its analogues, just as in Example 1.

Hoechst staining preliminarily validated the effects of SAC and its analogues on inhibiting apoptosis (Figure 10). Apoptosis is of great damage to cardiac structure and function, and is one of the major reasons for cardiac failure. The experimental results show the effects of SAC and its analogues on inhibiting myocardial cell apoptosis, and SAC and its analogues have great value in use of treating heart diseases.

### Example 6: synthesis of SPRC

This example is shown in Figure 11.

L-cysteine hydrochlorate was dissolved in a pre-cooled NH₄OH solution (2M, 240ml), and 3-propargyl bromide (14.5g, 0.124mol) was added to said solution; the mixed solution was stirred thoroughly at 0°C for 2 hours and filtered, then the filtrate was distilled in reduced pressure ( <40□) and concentrated to a smaller volume, and then filtered again. The solid separated out was washed repeatedly with ethanol, dried in vacuum and re-crystallized from water/ethanol with a 2:3 volume ratio to yield white needle crystals. The NMR structure was detected by Proton Nuclear Magnetic Spectroscopy (Figure 11).

### Example 7: experiments to validate effects of SPRC on myocardial infarction size

This example is shown in Figure 12 and Figure 13.

SPRC was dissolved in physiological saline solution. Male SD rats whose weights were in the range of 200-250g were randomly assigned to groups:
sham operated group (Sham, physiological saline solution, ip. n=4);
sham operatd+SPRC group (Sham+SPRC, 50mg/kg/day, n=4);
sham operatd+SAC group (Sham+SAC, 50mg/kg/day, n=4), model group (MI, physiological saline solution, ip. n=8);
model+SPRC group (MI+SPRC, 50 mg/kg/day, n =8);
model+SAC group (MI+SAC, 50 mg/kg/day, n =8).

Rats were pre-administrated with medicaments for seven days (ip.). On day 8, the rats were anesthetized by intraperitoneally injected 7% choral hydrate (5ml/Kg), and then were placed in a supine position, and their chests were prepared by shaving. A left thoracotomy was performed and the third intercostal space was exposed. The left anterior descending coronary artery was permanently ligated at the proximately 2-3mm from the origin between the left auricle and the cone of pulmonary artery with a 6-0 silk sature. The model was considered to be successfully established when area of myocardium supplied with blood turned to pallor and the ST segment was elevated as shown in the electrocardiogram of standard lime leads II lead. The chests were closed promptly and the skins were sutured. The rats were kept warm, given water and standard food and cultured in different cages after they were awake from anesthesia. Rats in sham operated group were prepared in the same manner except that the coronary artery was not ligated. The rats were administrated medicaments continuously for 2 more days. During the 48 hours after surgery, the changes in electrocardiogram were recorded. Blood samples were taken from abdominal aorta, then the rats were sacrificed and their hearts were taken promptly, put into 0.1 % TTC solution at pH 7.4 and incubated at 37°C for 15 minutes. The myocardial infarction sizes were observed after staining.

Compared with the model group, SPRC and SAC can obviously reduce myocardial infarction sizes *p* < 0.01 ( Figure 12). After 48 hours, the elevation of ST segment has some resumption (Figure 13). SAC and SPRC have a similar effect, *p*>0.05.

Table 1 shows the effect of SPRC on myocardial infarction size.

**Table 1**

| | Model group | SPRC | SAC |
|---|---|---|---|
| Infarction area/left ventricle area | 36.2±1.3 | 20.8±2.4 | 24.9±1.9 |

### Example 8: experiments to validate effects of SPRC on LDH leaking rate, and CK concentration, MDA and SOD in plasma

This example is shown in Figure 14.

Model rats of left coronary artery ligation were assigned randomly into sham operated group, sham operated+SPRC group, sham operated + SAC group, model group, model+SPRC group, model+SAC group (MI +SAC, 50 mg/kg/day, n =8), just as in Example 7.

It was observed from the experiments described above that LDH, CK activity and MDA content in the plasma of model group were obviously improved, whereas the SOD activity was lowered. When the cardiac tissue is injured by infarction, oxygen free radicals will be produced in a large amount, LDH shows that the integrity of myocardial membrane is damaged which then induces the leakage of LDH and CK; and MDA shows the peroxidization of the lipid in the myocardial membrane which then induces peroxidates to enter into blood; so monitoring SOD can indirectly indicate the ability of the body to clear away oxygen free radicals. The results show that both SPRC and SAC can obviously reduce the leakage of LDH and CK after myocardial infarction, inhibit the increase of MDA and efficiently protect SOD activity, demonstrating their capacity of reversing lipid peroxidization induced by blood deprivation and relatively strong ability to scanvage oxygen free radicals (Figure 14).

SPRC and SAC can decrease LDH leakage rate, lower the CK concentration and inhibit the production of MDA in plasma, as well as increase the SOD level in plasma.

Table 2 shows the effect of SPRC on LDH, CK activity, MDA content and SOD activity in plasma.

**Table 2**

| | LDH (U/L) | CK (U/ml) | MDA (nmol/ml) | SOD (U/ml) |
|---|---|---|---|---|
| sham operatd group | 2573±206 | 0.249±0.047 | 3.63±0.41 | 156.8±3.4 |
| sham operated+SPRC group | 2612±189 | 0.251±0.032 | 3.79±0.40 | 161.2±3.8 |
| sham operated+SAC group | 2599±212 | 0.250±0.019 | 3.57±0.31 | 159.8±4.8 |
| model group | 4641±251# | 0.502±0.055# | 5.78±0.61# | 91.3±2.3 |
| model+SPRC group | 3096±231** | 0.322±0.052* | 4.43±0.51 | 113.8±1.5 |
| model+SAC group | 3556±291* | 0.322±0.010* | 3.86±0.35* | 107.1±5.4 |

### Example 9: experiments to validate effects of SPRC on H₂S in plasma and enzyme CSE in cardiac tissue

This example is shown in Figure 15.

Model rats of left coronary artery ligation were assigned randomly into sham operated group, sham operated+SPRC group, sham operated+SAC group, model group, model+SPRC group, model+SAC group (MI +SAC, 50 mg/kg/day, n =8), just as in Example 7.

H₂S concentration in SPRC group was significantly increased compared with that in model group (Figure 15A), indicating the endogenous production of H₂S. The change of CSE activity in cardiac tissue was in the same trend as that of H₂S concentration in plasma, wherein the CSE activity in SPRC group was significantly increased compared with that in model group (*p*<0.01), and higher than that in SAC group with a significant difference (*p*<0.05)( please refer to Figure 15B).

According to literature reports, H₂S has the effect of removing active oxygen, so it has been speculated that the protection of SPRC and SAC on cardiac tissue after myocardial infarction is mediated through endogenously producing H₂S.

Table 3 shows the effects of SPRC on H₂S in plasma and enzyme CSE in cardiac tissue.

**Table 3**

| | H₂S concentration in plasma (µM) | CSE activity (µM /mg prot) |
|---|---|---|
| sham operated group | 60.3±5.3 | 2.01±0.14 |
| sham operated+SPRC group | 67.2±6.4 | 2.41±0.12 |
| sham operated+SAC group | 65.2±4.4 | 2.31±0.24 |
| model group | 34.7±5.6# | 1.58±0.15# |
| Model+SPRC group | 91.6±7.6* | 2.58±0.07* |
| model+SAC group | 61.1±3.6* | 2.19±0.32* |

### Example 10: experiments to validate effects of SPRC on increasing the expression of Bcl-2 and CSE at protein level and decrease the expression of Bax at protein level

This example is shown in Figure 16.

Model rats of left coronary artery ligation were assigned randomly into sham operated group, sham operated+SPRC group, sham operated+SAC group, model group, model+SPRC group, model+SAC group (MI +SAC, 50 mg/kg/day, n =8), just as in Example 7.

The protein expression level of Bax in Sham group was relatively low, and there was no significant difference on Bax and Bcl-2 between Sham group and Sham+SPRC and Sham+SAC groups; ligation of left coronary artery has led to the increase of Bax protein expression in the left ventricle, the decrease of Bcl-2 expression with a significant difference (*p*<0.05); compared with the MI group, there was a significant increase of Bcl-2 protein expression and significant decrease of Bax protein expression in SPRC group and SAC group (*p*<0.05). Compared with the MI group, the CSE protein expression was significantly increased in SPRC group and SAC group, and SPRC had an apparent effect on CSE (*p*<0.01, *p*<0.05). SPRC decreased the expression of apoptosis related factor Bax at protein level, and increased the expression of the anti-apoptosis related factors Bcl-2 and CSE at protein level (please refer to Figure 16).

### Example 11: experiments to validate that SPRC can decrease the expression of Bax at gene level, increase the expression of CSE at gene level and has no apparent effect on the gene expression of Bcl-2

This example is shown in Figure 17.

Model rats of left coronary artery ligation were assigned randomly into sham operated group, sham operated+SPRC group, sham operated+SAC group, model group, model+SPRC group, model+SAC group (MI+SAC, 50mg/kg/day, n =8), just as in Example 7.

There was no significant difference on Bcl-2 gene expression when SPRC and SAC were compared to Sham group and MI group (*p*<0.01); the gene expression of Bax in MI group was significantly improved compared with that in Sham group (*p*<0.01); Bax gene expression in SPRC group and SAC group was apparently lowered compared with that in MI group (*p*<0.05); compared with MI group, CSE gene expression in SPRC group was significantly increased (*p*<0.01), and CSE gene expression also had an obvious increse in SAC group, but less apparent than the effect of SPRC on CSE (*p*<0.05), indicating that SPRC has regulated down the expression of apoptosis related gene Bax at molecular level, and regulated up the expression of CSE at gene level (Figure 17).

### Example 12: experiments to validate that SPRC can increase the survival rate of myocardial cell line H9c2 injured by H₂O₂ and lower the LDH leaking rate of H₂O₂-injured myocardial cell H9c2.

This example is shown in Figure 18.

H9c2 was cultured in DMEM containing 10% fetal bovine serum in a 370 ,5 % CO₂ incubator. The cells were passaged when the confluence on monolayer was 90%. Adjust the density of cells to 5x10⁻⁴/well and inoculate the cells on a 96 well plate. The cells were divided into:
normal control group (Control): no medicament intervenation, no glucose and oxygen deprivation;
model group (Model): no medicament intervenation, under 200µmol/ L H₂O₂ for 2 hours; PAG group (PAG): PAG 10⁻⁴mol/L;
groups subjected to different dosage of SPRC: SPRC 1E-7, SPRC 1E-6 and SPRC 1E-5 were respectively subjected to 10⁻⁷, 10⁻⁶ and 10⁻⁵mol/L SPRC administration. SPRC+PAG group (SPRC+PAG): SPRC 10⁻⁵mol/L+PAG10⁻⁴mol/L.

Cell survival rate was assessed by MTT method. The results (Figure 18A) show that the survial rate in model group was significantly lower than that in normal control group, SPRC medium dosage group and high dosage group can significantly increase the survival rate (*p*<0.05). PAG was able to block the cell protection effect of SPRC.

10⁶ cells from each sample of each group (n = 4) were lysed thoroughly, and LDH contents in cells were tested by pyruvic acid method. As shown in Figure 18B, the results illustrate that LDH leaking rates in groups subjected to different dosages of SPRC were significantly lowered compared with that in model group, and PAG could significantly block the SPRC protective effect, indicating that the effect of SPRC on heart and blood vessels protection was correlated with the endogenous production of H₂S at least in part.

Table 4 shows the results that SPRC increased survival rate of myocardial cell line H9c2 injured by H₂O₂ and lowered LDH leaking rate of H₂O₂ injured myocardial cell H9c2.

**Table 4**

| | Cell Survival rate (%) | LDH leaking rate (%) |
|---|---|---|
| normal control group | 100.0±2.4 | 0.0±3.6 |
| Model group | 59.4±0.9# | 54.2±0.9# |
| PAG group | 58.9±1.2 | 63.4±1.2 |
| SPRC low dosage group | 62.4±1.6 | 51.4±1.8 |
| SPRC medium dosage group | 64.1±1.5* | 50.0±2.6* |
| SPRC high dosage group | 69.1±1.1* | 43.9±3.0* |
| SPRC+PAG group | 60.9±0.3& | 60.6±1.9& |

### Example 13: experiments to validate that SPRC can improve the activity of SOD and inhibit the production of lipid peroxidation product MDA

This example is shown in Figure 19.

H₂O₂-injured myocardic cell line H9c2 were randomly divided into normal control group (Control), model group (Model), PAG group (PAG), and SPRC low, medium and high dosage groups, just as in Example 12.

The results show (Figure 19A) that SPRC medium and high dosage groups both can apparently improve the activity of Mn-SOD, which were significantly increased compared with that in model group (*p*<0.05). This effect could be blocked by PAG, indicating that the effect of SPRC on significanlty improving the activity of Mn-SOD was correlated with the production of H₂S at least in part.

The MDA content in model group was significantly increased compared with that in normal control group (Figure 19B) (*p*<0.05); in SPRC low, medium and high dosage groups were obviously decreased than model group, demonstrating SPRC can inhibit the lipid peroxidation induced by active oxygen and the effect can be blocked by PAG

Table 5 shows the results of SPRC on improving SOD activity and inhibiting the production of lipid peroxidation product MDA.

**Table 5**

| | Mn-SOD activity (U/mgprot) | MDA content (nmol/mgprot) |
|---|---|---|
| normal control group | 9.23±0.19 | 1.25±0.12 |
| model group | 8.46±0.66 | 2.28±0.10# |
| PAG group | 7.57±0.83 | 2.71±0.26 |
| SPRC low dosage group | 8.75±0.68 | 1.89±0.09 |
| SPRC medium dosage group | 9.89±0.83* | 1.70±0.08* |
| SPRC high dosage group | 12.45±1.63* | 1.42±0.04* |
| SPRC+PAG group | 8.78±0.74& | 2.21±0.19& |

### Example 14: experiments to validate the effect of SPRC on inhibiting myocardial cells apoptosis

This example is shown in Figure 20.

H₂O₂-injured myocardic cell line H9c2 were randomly divided into normal control group (Control), model group (Model), PAG group (PAG), and SPRC low, medium and high dosage groups, just as in Example 12.

Hoechest and PI double-staining validated the inhibition effect of SPRC on apoptosis (Figure 20). Apoptosis is of great damage to cardiac structure and function, and is one of the major reasons for cardiac failure. This experiment validated the anti-apoptosis effect of SPRC in the whole animal model from both gene and protein level, and confirmed the effect of SPRC on H9c2 myocardial apoptosis induced by H₂O₂ without the intervention of other factors by Hoechest and PI double-staining method at cell level, validating the great value of SPRC in use of treating heart diseases.

## Claims

1. A use of a compound of formula I as follows in the preparation of medicaments for preventing and/or treating myocardial injury: 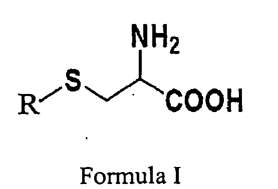 wherein, R is selected from the group consisting of linear or branched alkyl of C₁-C₅, alkenyl and alkynyl of C₁-C₅, and preferably terminal olefine and terminal alkyne; and said compound of formula I is present in the form of the compound itself, its pharmaceutically acceptable salts or its solvates.

2. The use according to claim 1, wherein said heart disease caused by myocardial injury is a heart disease caused by hypoxia and hypoglycemia-injured myocardial injury.

3. A use of compound of formula I as follows in preparation of medicaments having the effects of increasing cell survival rate, producing endogenous H₂S, reducing LDH leakage of myocardial cells injured by hypoxia and hypoglycemia, improving the activity of SOD and/or Catalase, improving the ability of tissue to inhibit production of hydroxyl free radicals, inhibiting lipid peroxidation and/or inhibiting myocardial cells apoptosis: 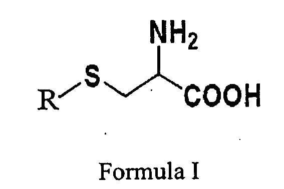 wherein, R is selected from the group consisting of linear or branched alkyl of C₁-C₅, alkenyl and alkynyl of C₁-C₅, and preferably terminal olefine and terminal alkyne; and said compound of formula I is present in the form of the compound itself, its pharmaceutically acceptable salts or its solvates.

4. A use of compound of formula I as follows, its pharmaceutically acceptable salts or its solvates in preparation of medicaments having the effects of reducing myocardial infarction size, decreasing the release of lactate dehydrogenase and creatine kinase, improving the activity of superoxide dismutase, lowering the level of malondialdehyde, endogenously producing H₂S and/or inhibiting apoptosis: 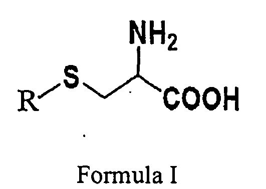 wherein, R is selected from the group consisting of linear or branched alkyl of C₁-C₅, alkenyl and alkynyl of C₁-C₅, and preferably terminal olefine and terminal alkyne.

5. The use according to any of claims 1 to 4, wherein said compound of formula I is selected from the group consisting of S-allyl cysteine, S-ethyl cysteine, S-propyl cysteine, S-allylmercapto cysteine, S-butyl cysteine, S-pentyl-L-cysteine and S-propargyl cysteine, preferably S-allyl cysteine and S-propargyl cysteine, and most preferably S-allyl cysteine.

6. The use according to any of claims 1 to 5, wherein said dosage form of said medicaments is selected from the group consisting of formulations for oral, parenteral, topical, inhalation and transdermal administration.

7. A pharmaceutical composition for preventing and/or treating myocardial injury, wherein said pharmaceutical composition comprises:
compound of formula I, its pharmaceutically acceptable salts or its solvates as the active ingredient: 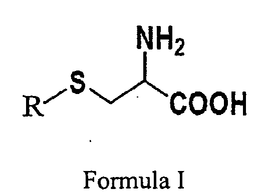
wherein, R is selected from the group consisting of linear or branched alkyl of C₁-C₅, alkenyl and alkynyl of C₁-C₅, and preferably terminal olefine and terminal alkyne;
and one or more pharmaceutically acceptable carriers.

8. The pharmaceutical composition according to claim 7, wherein said solvates of compound of formula I are hydrates.

9. The pharmaceutical composition according to claim 7 or 8, wherein said compound of formula I is selected from the group consisting of S-allyl cysteine, S-ethyl cysteine, S-propyl cysteine, S-allylmercapto cysteine, S-butyl cysteine, S-pentyl cysteine and S-propargyl cysteine, preferably S-allyl cysteine and S-propargyl cysteine, and most preferably S-allyl cysteine.

10. The pharmaceutical composition according to any of claims 7 to 9, wherein said pharmaceutical composition is selected from the group consisting of formulations for oral adminsitration, parenteral administration such as injection, topical administration, inhalation administration and transdermal administration.

11. The pharmaceutical composition according to claim 10, wherein said pharmaceutical composition is selected from the group consisting of oral formulations of tablets, capsules, granules, pills, drops, juice or syrup; preferably, said pharmaceutically acceptable carrier is selected from the group consisting of disintegrating agents, lubricants, binders, filling agents, solvents, flavours, sweetening agents, antioxidants, surfactants, preservatives, correctants and pigments.

12. A method for preparation of the compound of formula I as follows: 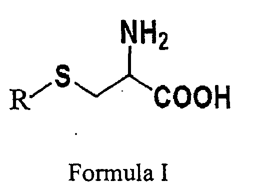 wherein, R is selected from the group consisting of linear or branched alkyl of C₁-C₅, alkenyl and alkynyl of C₁-C₅, and preferably terminal olefine and terminal alkyne;
said method comprises steps as follows:
L-cysteine hydrochloride is dissolved in a pre-cooled NH₄OH solution, and Br-R is added to said solution, wherein the substituent R is defined as aforementioned; the mixed solution is stirred at 0-5□ for 1-5 hours and filtered, then the filtrate is distilled in reduced pressure, concentrated and then filtered again. The solid separated out is washed repeatedly with ethanol, dried in vacuum and re-crystallized from water/ethanol with a volume ratio from 1: 3 to 3:1.
